# EUROPEAN PATENT APPLICATION

(11) **EP 2 305 216 A1**
(43) Date of publication of application: **06.04.2011**
(21) Application number: 09171396.6
(22) Date of filing: 25.09.2009
(51) Int. Cl.: A61K 9/127

(54) **Two step ultrasound protocol for drug delivery**

(71) Applicant: Koninklijke Philips Electronics N.V., 5621 BA Eindhoven (NL)
(72) Inventor: The designation of the inventor has not yet been filed
(74) Representative: Damen, Daniel Martijn

(57) **Abstract**

Described is a method for the delivery of a bio-active molecule to a mammal comprising the steps of; (i) administrating a temperature sensitive carrier comprising a bio-active molecule; (ii) using a first ultrasound pulse so as to release the bio-active molecule from the temperature sensitive carrier, and (iii) using a second ultrasound pulse so as to facilitate sonoporation and subsequent intracellular uptake of the bio-active molecule, wherein step (iii) can be performed before, and/or after steps (i) to (ii). The method according to the invention is applicable to a broad range of bio-active molecules such as nucleotide based drugs (e.g. siRNA, mRNA, antisense, plasmid DNA) or small molecules that otherwise would not cross the cell membrane, including peptides and small proteins.

## Description

### FIELD OF THE INVENTION

The invention relates to a method for the delivery of a bio-active molecule to a mammal by using ultrasound and a composition comprising a thermosensitive carrier comprising a bio-active molecule and an ultrasound active particle. The invention further relates to a method of treatment of a tumor in a patient.

### BACKGROUND OF THE INVENTION

Current drug delivery vehicles for biologicals or nucleotide-based drugs are engineered to deliver their payload to target organs. If the drug payload has to be released inside a cell (e.g. cytosolic delivery), sophisticated engineering of the drug delivery carrier is required to ensure the drug to be released intracellular. Normally, drug delivery carriers are taken up by endocytic pathways and end up inside the cell within endosomes that prohibit efficient drug release into the cytosol. The direct delivery of the drug of interest across the cell membrane into the cytosol would be highly beneficial.

Ultrasound offers an alternative to complex biological process for controlling the release, delivery or activation of therapeutic agents such as DNA. Certain carrier vehicles such as ultrasound active particles and nano-droplets, can be triggered by suitable ultrasound to release their contents. In case of gene delivery, mixing bubbles with the nucleotide can be sufficient to achieve drug efficacy. The most likely mechanism for such effects is that since the micro-bubbles oscillate in the ultrasound field, microscopic fluid motions drive the therapeutic agents into cells or temporarily open pores in the cell membrane allowing free movement of molecules. It is also known that ultrasound in the presence of microbubbles can open the blood-brain barrier, allowing drugs to reach the target cells in the brain.

An example of the use of ultrasound for DNA targeting is shown in Huber et al, where naked DNA is transfected into a carotid artery by using focused ultrasound. (Nature 457, 405-412 (22 January 2009).

### SUMMARY OF THE INVENTION

It would be advantageous to provide a method of ultrasound-mediated delivery of therapeutic agents that would allow an enhanced targeting accuracy and drug release efficiency.

In order to address this objective the invention presents a method for the delivery of a therapeutic agent into a mammal comprising the steps of:
- administrating a temperature sensitive carrier comprising a bio-active molecule;
- using a first ultrasound pulse so as to release the bio-active molecule from the temperature sensitive carrier, and
- using a second ultrasound pulse so as to facilitate sonoporation and subsequent intracellular uptake of the bio-active molecule, wherein step (iii) can be performed before, and/or after steps (i) to (ii).

The method according to the invention is applicable to a broad range of bioactive molecules such as nucleotide based drugs (e.g. siRNA, mRNA, antisense, plasmid DNA) or small molecules that otherwise would not cross the cell membrane, including peptides and small proteins. This invention discloses a new strategy to deliver drugs into the cell using a two-step process. The bio-active molecule is enclosed in a temperature sensitive carrier, and can be released by the application of focused ultrasound. Additionally, the site of release is subjected to ultrasound with a more stringent protocol to locally induce sonoporation. This leads to controlled release of a biological agent, and subsequent efficient incorporation of this bio-active agent into the cell. The second ultrasound pulse can also be used to facilitate increased extravasion of the carrier.

In a preferred embodiment, an ultrasound active particle is administrated before step (iii). This reduces the ultrasound pressure that is needed to facilitate sonoporation and or etravasion of the thermosensitive carrier.

In a more preferred embodiment, the ultrasound active particles and the thermosensitive carriers are administered simultaneously to reduce patient inconvenience.

In yet another preferred embodiment, the temperature sensitive carrier is a low temperature sensitive carrier with a release temperature between 39 and 44 degrees Celsius. This facilitates the release of the bio-active agent from the temperature sensitive carrier at a physiologically acceptable temperature range.

In another embodiment, the temperature sensitive carrier is a liposome. Even more preferably, this liposome is PEGylated. This leads to increased circulation times of the liposomes and therefore to an optimized treatment efficiency.

Preferably, the biologically active molecule is a nucleic acid.

The invention further relates to a method of treatment of a tumor in a patient, comprising the targeted delivery of an anti-tumor agent to the site of the tumor, wherein the targeted delivery is conducted by applying a method according to any the invention.

The invention further relates to a composition for the delivery of a bio-active molecule to a mammal comprising:
- a temperature sensitive carrier comprising a bio-active molecule
- an ultrasound active particle
- a suitable injection fluid

The administration of Ultrasound active Particles and temperature sensitive carriers typically is by intravenous administration, although other types of administration are not excluded (e.g. application into a body cavity or intra-arterial or intra-lymphatic system).

### FIGURES

Fig. 1: Ultrasound mediated drug delivery using temperature-sensitive carriers in a 2 step protocol
Fig. 2: release of TO-PRO-3 from temperature sensitive liposomes
Fig. 3: intracellular delivery of TO-PRO-3 using a two-step ultrasound protocol
Fig 4-7: drug delivery protocols according to the present invention

### DETAILED DESCRIPTION OF THE INVENTION

The present invention will be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

### Ultrasound

Ultrasound is well-known in the art as acoustic energy that can be applied for imaging (e.g. fetuses in the womb). Increasingly, ultrasound is also described as a source of external energy that can affect drug release, by affecting physical properties of ultrasound-sensitive carriers.

Ultrasound is generally applied by means of a transducer probe that sends (and receives) ultrasonic sound waves. When using ultrasound to activate drug delivery, the basic requirement is that ultrasonic waves can be transmitted into a person's body (i.e. the receiving is of secondary importance here). In carrying out the invention, a therapy applicator is thus provided that serves to transmit ultrasonic waves into a person's body. Such applicators are known, as described e.g. in WO 2006/131840.

According to the present invention, it is required that the ultrasound transducer probe is capable of generating at least two different intensities or pressures. It should be able to facilitate the activation of temperature sensitive carriers by increasing the temperature at least to the release temperature of the carriers. Furthermore, it should be able to locally facilitate the sonoporation of tissue leading to the intracellular uptake of the bio-active agent that is released from the temperature sensitive carrier.

The minimum and maximum ultrasound energy levels (including center frequency, amplitude, pulse length as well as pulse repetition, frequency, or frame rate) required for local heating and sonoporation are determined by the size and composition of both ultrasound active particles and temperature sensitive carrier. The local drug release rate may be adjusted by tuning the ultrasound energy level and thereby the temperature increase obtained in the tissue.

### Ultrasound active particles

This generally refers to particles such as microbubbles, microparticles, nanoparticles, microcapsules or nanocapsules, having in common that they undergo a physical change upon the application of ultrasound. This change can effect e.g. the particles' physical state (e.g. they can melt) or integrity (e.g. ultrasound-mediated destruction of microbubbles) or shape/size (e.g. they can oscillate in size) or porosity (e.g. they can make their payload temporarily available to the surrounding medium).According to the present invention, the ultrasound active particles are used to induce sonoporation.

Particles capable of activation by ultrasound (hereinafter referred to as Ultrasound active Particles) include aqueous suspensions of gaseous microbubbles. These exhibit desirably large differences in acoustic impedance between a gas (e.g., air) and the surrounding aqueous medium. A size requirement for these microbubbles exists; they must be injectable intravenously and be small enough to pass through the capillaries of most tissues; i.e., they have to be smaller than about 8 microns. However, very small microbubbles (ca. 1 micron) are not very echogenic, and a compromise has to be made. To possess high echogenicity but still pass through the capillaries of most tissues, 3-4 microns are considered to be an optimal size. In addition, the size of the particle controls the resonance and or rupture of the particle, and therefore 2 to 4 micron diameter particles are preferably chosen. An optimal sonoporation frequency lies in the frequency range of 1 to 10 MHz.

### Temperature sensitive carriers

The invention preferably provides for carriers that are thermosensitive. This means that the physical or chemical state of the carrier is dependent on its temperature. These thermosensitive carriers have an average size between 50-1000 nm, preferably between 50-400 nm and even more preferably between 50-200 nm

Any thermosensitive carrier that can package a molecule of interest and that is intact at body temperature (i.e. 37°C) but destroyed at any other, non-body temperature that can be tolerated by a subject may be used. Temperature sensitive carriers according to the invention include but are not limited to thermosensitive micro- and nanoparticles, thermosensitive polymersomes, thermosensitive liposomes, thermosensitive nanovesicles and thermosensitive nanospheres.

Thermosensitive liposomes include but are not limited to any liposome, including those having a prolonged half-life, e.g. PEGylated liposomes.

Liposomes are generally spherical vesicles comprising a bilayer membrane enclosing a cavity, or lumen. The bilayer can be made up of at least one phospholipid and may or may not comprise cholesterol. Liposomes can be composed of naturally-derived phospholipids with mixed lipid chains (like egg. phosphatidylethanolamine), or of pure surfactant components like dioleoylphosphatidylethanolamine (DOPE). The term liposomes, as used in the description of the invention, includes lipid spheres usually denoted micelles.

Thermosensitive carriers for use in the invention ideally retain their structure at about 37°, i.e. human body temperature, but are destroyed at a higher temperature, preferably only slightly elevated above human body temperature, and preferably also above pyrexic body temperature. Typically about 42°C is a highly useful temperature for thermally guided drug delivery.

The required heat to raise the temperature of the thermosensitive drug carriers so as to promote the destruction of the thermosensitive carriers may be used. Heat can be applied by using a focused energy source capable of inducing highly localized hyperthermia.

Thermosensitive liposomes are known in the art. Liposomes according to the present invention may be prepared by any of a variety of techniques that are known in the art. See, e.g., U.S. Pat. No. 4,235,871; Published PCT applications WO 96/14057; New RRC, Liposomes: A practical approach, IRL Press, Oxford (1990), pages 33-104; Lasic, D.D., Liposomes from physics to applications, Elsevier Science Publishers, Amsterdam, 1993; Liposomes, Marcel Dekker, Inc., New York (1983).

Entrapment of a drug or other bio-active agent within carriers of the present invention may also be carried out using any conventional method in the art. In preparing carrier compositions of the present invention, stabilizers such as antioxidants and other additives may be used as long as they do not interfere substantially with the purpose of the invention.

In the foregoing, reference is made to a thermosensitive carrier suitable for the localized delivery of a biologically active agent, such as a drug. Hereinafter, the term "biologically active agent" will be referred to, in short, as "drug".

A drug is a chemical substance used in the treatment, cure, prevention, or diagnosis of a disease or disorder, or used to otherwise enhance physical or mental well-being. The guided delivery foreseen with the present invention will mostly be useful therapeutic agents (i.e. drugs in a strict sense, intended for therapy or prevention of diseases or disorders), but also for agents that are administered for diagnostic purposes. Although other bio-active agents, i.e. those that are not therapeutic or diagnostic, such as functional food ingredients, will not generally be subjected to guided and/or monitored delivery, such could be done using the present invention if desired.

The most optimal use of the invention is attained in the case of targeted intracellular therapeutics; so drugs that are intended for targeted delivery and that cannot pass the cell membrane by passive means. This pertains to agents to be delivered intracellulary, e.g. in the treatment of tumors, in the treatment or prevention of cardiovascular disorders, such as atherosclerosis in the coronary arteries, or to antithrombotic agents (e.g. for locally resolving blood cloths) or agents that require passing the blood-brain barrier such as neuromodulators as can be used in the treatment of neural conditions such as epilepsy, Alzheimer's disease, Parkinson's disease, or stroke.

Bio-active agents suitable for use in the present invention include biologically active agents including therapeutic drugs, endogenous molecules, and pharmacologically active agents, including antibodies; nutritional molecules; cosmetic agents; diagnostic agents; and additional contrast agents for imaging. As used herein, an active agent includes pharmacologically acceptable salts of active agents.

The drug carriers of the present invention can comprise either hydrophilic or hydrophobic bioactive agents. A hydrophilic bioactive agent could be encapsulated in the aqueous compartment of the carrier, whereas hydrophobic bioactive agents could be incorporated in hydrophobic domains of the carrier, for instance in the lipid bilayer of liposomes. Nucleic acids, carbohydrates and, in general, proteins and peptides are water soluble or hydrophilic. For instance, bioactive agents which are small molecules, lipids, lipopolysaccharides, polynucleotides and antisense nucleotides (gene therapy agents) are also envisaged. Such biologically active agents, which may be incorporated, thus include non-peptide, non-protein drugs. It is possible within the scope of the present invention to incorporate drugs of a polymeric nature, but also to incorporate drugs of a relatively small molecular weight of less than 1500 g/mol, or even less than 500 g/mol.

Accordingly, compounds envisaged for use as bioactive agents in the context of the present invention include any compound with therapeutic or prophylactic effects. It can be a compound that affects or participates in tissue growth, cell growth, cell differentiation, a compound that is able to invoke a biological action such as an immune response, or a compound that can play any other role in one or more biological processes.

The drug may be present in the inner, the outer, or both of the compartments of the carrier, e.g. in the cavity and/or in the shell of a liposome. The distribution of the drug is independent of the distribution of any other agents comprised in the drug carrier, such as a paramagnetic chemical shift reagent or a paramagnetic agent. A combination of drugs may be used and any of these drugs may be present in the inner, the outer, or both of the compartments of the drug carrier, e.g. in the cavity and/or in the shell of a liposome

### Treatment protocol

Temperature-sensitive carriers release their content upon heating using an external trigger, in particular focused ultrasound. Focused ultrasound allows to heat tissue non-invasively in a controlled and well-defined fashion. The technology is available, for instance, under MR image guidance or ultrasound imaging. In the delivery process a number of distinct steps can be distinguished. In short, these steps comprise the release of a bio-active molecule from a thermosensitive carrier and the sonoporation of tissue or cells to facilitate the intracellular uptake of the bio-active molecule.

Sonoporation, preferably in the presence of ultrasound active particles, opens up the cell membranes allowing the entry of molecules that would otherwise not enter the cell. The extent of pore formation depends on the ultrasound settings which should be tuned to create pores that can be resealed again, but for efficient delivery preferable on a fairly long time scale. Injecting the thermosensitive carrier and optionally ultrasound active particles in a cell culture or intravenously, followed by the application of ultrasound is applicable in vitro and for the in vivo delivery of drugs to endothelial cells. Conditions for mild heating, performed at 1 MHz include for instance 3 MPa, continuous wave for 1 minute or 8-9 MPa at a duty cycle of 10% with 100 ms pulses under such conditions cavitation nuclei as ultrasound active particles will be activated but potentially not at the preferred conditions for resealable sonoporation.

For in vivo applications, the ultrasound settings should be chosen such that first cavitation is promoted, which is most efficient with pulsed ultrasound, while subsequently mild ultrasound heating can be applied to release the drug from the thermosensitive carrier.

Four examples (A-D) of treatment protocols according to the invention are disclosed below.

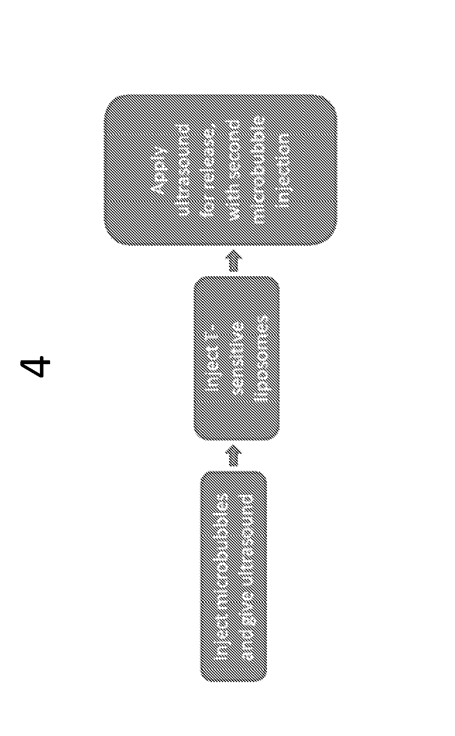

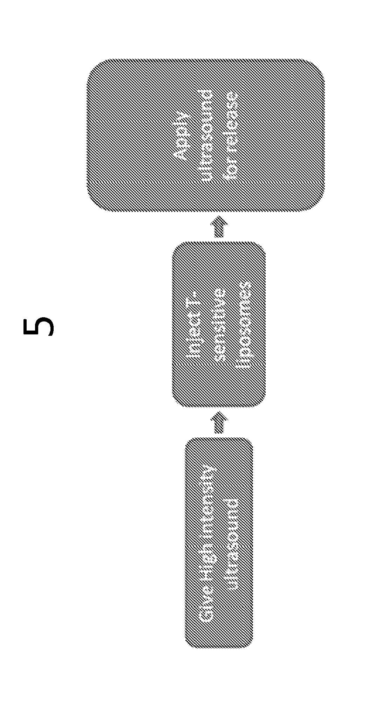

In protocols A (fig 4) and B (fig 5), the first step involves the accumulation of the temperature-sensitive drug carrier in the tissue of interest. This accumulation can be passive by diffusion of the temperature sensitive carrier, or enhanced by ultrasound, potentially in the presence of ultrasound active particles In the absence of ultrasound active particles, the ultrasound pressure to be used is for instance in the range of 8-9 MPa at a frequency of 1 MHz and a duty cycle of 5%. In the presence of ultrasound active particles the applied pressure can be decreased to 0.3-2.5 MPa. Alternatively, relatively long pulses, 10 ms and more, at frequencies between 0.5 and 2 MHz, can be applied. Using microbubbles or other ultrasound active particles to open up the vasculature has, in this case, the advantage that opening of the vessels can be established at relatively low acoustic pressure without the risk of prematurely heating the tissue by which release from the temperature sensitive system would already occur.

The next steps involve the creation of pores in the target cells and the release of the drugs from the temperature-sensitive to allow for cell uptake.

The release requires a temperature increase in the order of 4-5 degrees which is effectuated by pulsed ultrasound as described by Dromi et al: (1300 W/cm², 10% duty cycle, 100 ms pulses, duration 15-20 minutes).

An essential part of the invention is the use of sonoporation. In the presence of ultrasound active particles, pores in cells other than endothelial cells can be created as shown by a plasmid DNA transfection experiments known in the art in which "double pulse" schemes are used to transfect e.g. cardiomyocytes. It is possible to induce sonoporation also in other cells (apart from endothelial cells) using ultrasound and ultrasound active particles.

By following the protocols proposed, optimal conditions for the timing and treatments can be identified. In protocol ultrasound active particles are injected first, and because of their limited circulation time, exposed to ultrasound immediately as they enter the region of interest. Frequencies to be used are in the range of 0.5 to 3 MHz, the pressure between 0.3 and 3 MPa, the time in between pulses should be chosen such that refill of the tissue vasculature is possible, and is preferable in the order of 5 or more seconds. The number of length of the pulses can vary widely, long pulses in the order of 5000 -20,000 cycles are preferred. At higher pressures and longer pulse lengths temporary halting of blood supply to tumors in mice was observed.

Through the created pores the temperature-sensitive carriers extravasate, which can be injected directly after or even before as the used ultrasound conditions do not lead to heating. The time allowed for extravasation depends on the circulation time of the agent, preferably between 4 and 48 hours. At this time a second ultrasound active particle injection is given and ultrasound is applied again to create porosity also in the cells behind the endothelium. The same ultrasound parameters as in the first ultrasound active particle controlled sonoporation treatment can be used, followed by a treatment to heat of the tissue gently, which requires for instance continuous ultrasound or pulsed ultrasound at settings as indicated by Dromi et al. (9 MPa, 5% duty cycle, 100 ms pulses) or at lower pressure, in continuous wave mode for instance 3 MPa for about 1 minute. The heating protocol differs from the sonoporation protocol; cavitation is more efficient if pulsed ultrasound is given

In Protocol B no ultrasound active particles are used. First an ultrasound treatment is given, in a pulsed mode to avoid premature heating, with similar pulse lengths but a higher acoustic pressure of 3 to 10 MPa at the frequencies, duty cycles and pulse lengths as in protocol A to create cavitation. The number of cavitation nuclei in blood is higher than for instance in muscle tissue so in this step the main pore formation will still be in the vasculature. Through the created pores the temperature- sensitive carriers extravasate, which can be injected directly after or even before. The time allowed for extravasation depends on the circulation time of the agent and is, preferably, between 4 and 48 hours. At this time the ultrasound treatment is repeated. Obviously protocols can be chosen where ultrasound active particles are injected only once, in the first or only in the second step or both.

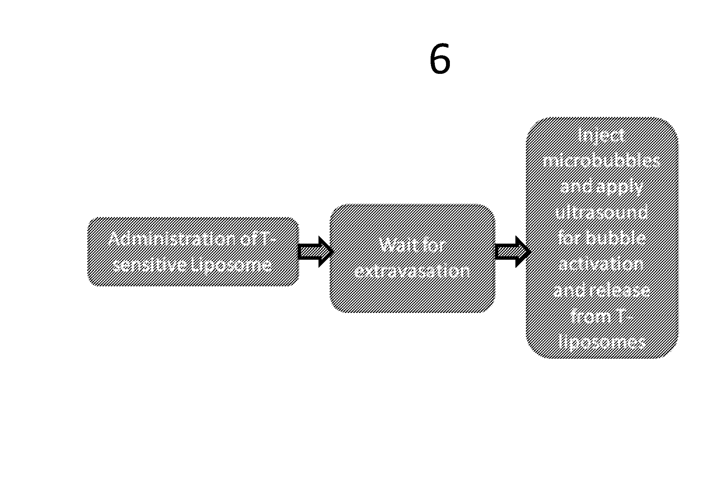

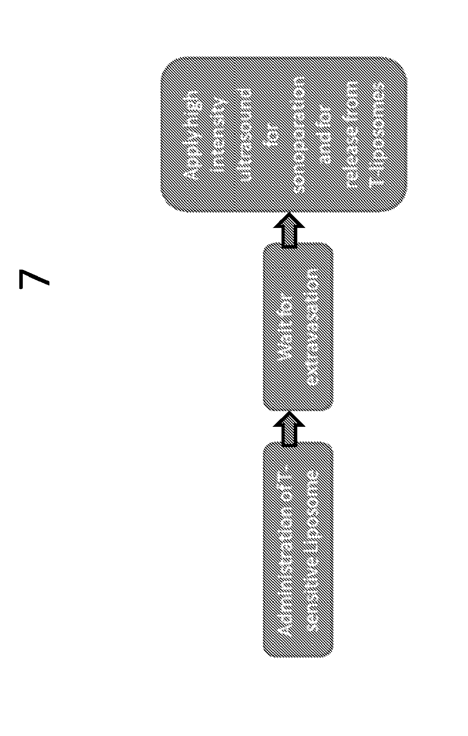

If natural extravasation of the temperature sensitive carrier is high, the first ultrasound treatment is not necessary and protocols C (fig 6) and D (fig 7) can be used. In protocol C the temperature sensitive carriers are injected followed by waiting for maximum extravasation for 4-24 or potential 48 hours. Subsequently ultrasound active particles are injected and the tissue is treated at the settings given in Protocol A. In protocol D the same procedure if followed but without injection of ultrasound active particles and ultrasound is given according to the settings given in Protocol D.

### Example

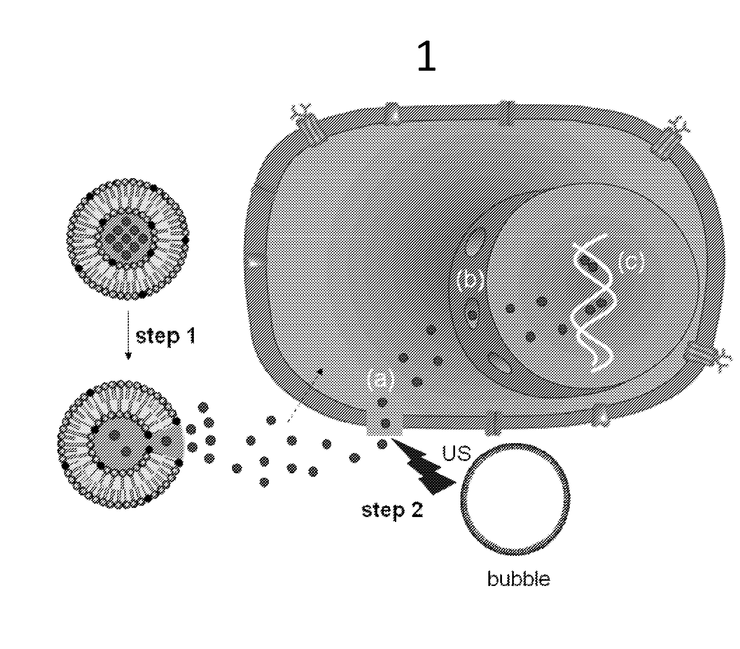

Temperature-sensitive liposomes were used for the local delivery of TO-PRO-3 (as a model drug) that acts on the DNA in the nucleus. It is important to note that TO-PRO-3 cannot cross the cell membrane of a living cell. TO-PRO-3 was encapsulated in the inner aqueous cavity of a temperature-sensitive liposome. The release of this dye from the liposomal carrier was achieved by increasing the temperature above the melting phase transition temperature (Tₘ) of the phospholipid membrane (Figure 1, step 1). Subsequently, ultrasound was induced supported by ultrasound active particles to induce sonoporation of the cell membrane (Figure 1, step 2). The oscillation and disruption of the ultrasound active particles caused cellular uptake of TO-PRO-3. The intercalation of TO-PRO-3 into the DNA of the cell nucleus induces a strong increase of the fluorescence, which was used to probe the cellular uptake of TO-PRO-3.

Temperature sensitive-liposomes were prepared using the lipid film hydration technique. The phospholipids; 1,2-dipalmitoyl-sn-glycero-3-phosphocholine (DPPC), 1-palmitoyl-sn-glycero-3-phosphocholine (MPPC) and 1,2-dipalmitoyl-sn-glycero-3-phosphoethanolamine-N-[methoxy(polyethyleneglycol)-2000] (DPPE-PEG2000) (86:10:4) were dissolved in a solution of chloroform/methanol (4:1 v/v). The solvent was removed in vacuo until a thin lipid film was obtained. The lipid film was further dried under a nitrogen flow overnight. The lipid film was hydrated at 60°C with HEPES buffered saline (HBS) (20 mM HEPES, 8% NaCl, pH 7.4), containing 10 µM TO-PRO-3 (TO-PRO-3® iodide, Invitrogen). The sample was extruded at 60°C, two times through a 400 nm filter, two times through a 200 nm filer and five times through a 100 nm filter. After extrusion, the extraliposomal TO-PRO-3 was removed by gel filtration using a PD-10 column (GE Healthcare), which was pre-equilibrated with HBS (pH 7.4). The melting phase transition temperature of the phospholipid membrane of 40.5°C was determined by means of differential scanning calorimetry. The release of TO-PRO-3 from the liposome in an aqueous solution containing DNA (10 mg/mL DNA from herring sperm, Sigma Aldrich) was studied during heating by probing the fluorescence at 661 nm (λₑₓ = 642 nm). The mean average hydrodynamic radius of the TO-PRO-3 containing TSLs was determined by means of dynamic light scattering. As a proof of concept for US-mediated drug delivery using temperature-sensitive liposomes as drug vehicles, C6 rat glioma cells were seeded in a NUNCTM OpticellTM cell culture system and incubated with TO-PRO-3-TSLs at 37°C. Focused US (6 mm single element transducer at 1.5 MHz; power of 1 W, pulse repetition frequency of 1 kHz, duty cycle of 20%, duration 3 min) was applied at some areas. Next, the Opticell was heated to 41°C and US was applied at some other areas with the same parameters. The final TO-PRO-3 concentration upon release in the Opticell was estimated to be 1 µm. Finally, ultrasound active particles (Sonovue, Bracco) were added and again US was applied at some areas. After every step, images were acquired using the epifluorescence microscope (Leica DM R equipped with a CCD camera and an appropriate set of filters).

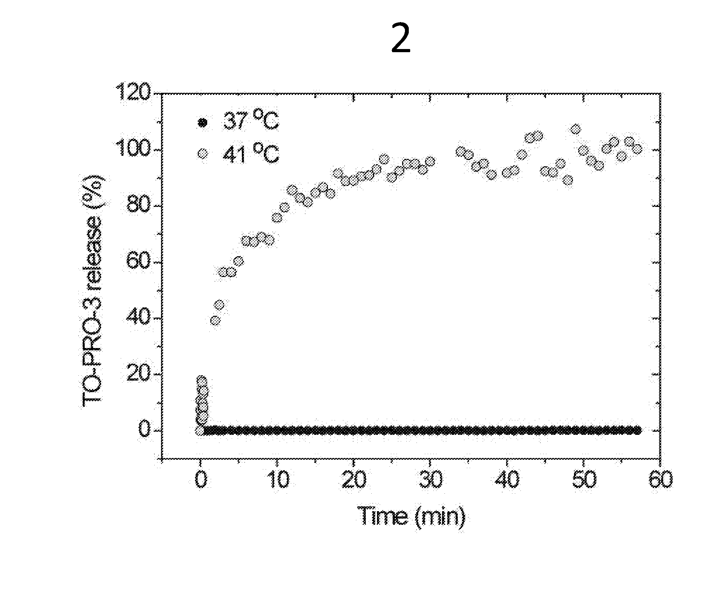

Figure 2 shows temperature-sensitive liposomes containing TO-PRO-3 in a solution containing DNA: the fluorescence intensity as a function of the temperature. At temperatures below the melting phase transition temperature (T < Tm), the intensity of the fluorescence of TO-PRO-3 was relatively low, whereas at T ≈ Tm a strong increase of the fluorescence was observed due to the release of TO-PRO-3 from the liposomal carrier followed by the intercalation of TO-PRO-3 into the DNA.

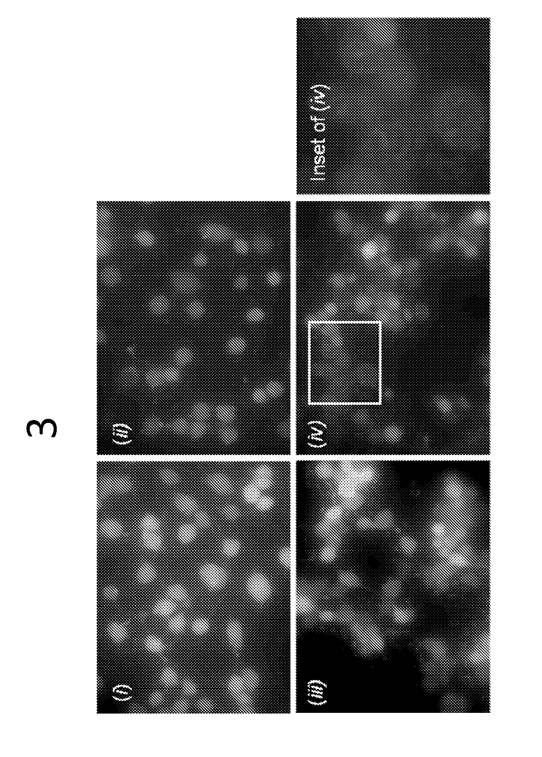

The intracellular delivery of TO-PRO-3 using a two-step protocol was studied using living, not permeabilized cells (Figure 3). In the first step, the release of TO-PRO-3 from the liposomal carrier was induced by increasing the temperature to 41°C (T > Tm). Figure 3 shows cells incubated with TO-PRO-3-TSLs: (i) after incubation at 37°C;(ii) after incubation at 41°C; (iii) after incubation at 41°C and the addition of Sonovue (without the application of US); (iv) after incubation at 41°C and the addition of Sonovue (with the application of US). After every step some areas were exposed to US. The inset (bottom right) shows the TO-PRO-3 nuclear staining in more detail. Only after the addition of Sonovue and the application of US, TO-PRO-3 bound to the DNA of the cells was observed. In addition, the morphology of the cells remained unaffected upon the application of US.

## Claims

1. A method for the delivery of a bio-active molecule to a mammal comprising the steps of :
(i) administrating a temperature sensitive carrier comprising a bio-active molecule;
(ii) using a first ultrasound pulse so as to release the bio-active molecule from the temperature sensitive carrier, and
(iii) using a second ultrasound pulse so as to facilitate sonoporation and subsequent intracellular uptake of the bio-active molecule, wherein step (iii) can be performed before, and/or after steps (i) to (ii).

2. Method according to claim 1, wherein an ultrasound active particle is administrated before step (iii).

3. Method according to claim 1 and 2 wherein the temperature sensitive carrier is a low temperature sensitive carrier with a release temperature between 39 and 44 degrees Celsius.

4. Method according to any one of claims 1 to 3, wherein the temperature sensitive carrier is a liposome.

5. A method according to any one of claims 1 to 4, wherein the low temperature sensitive carrier and the ultrasound active particle are administered simultaneously.

6. A method according to claim any one of claims 1 to 5, wherein the biologically active molecule is a nucleic acid.

7. A method of treatment of a tumor in a patient, comprising the targeted delivery of an anti-tumor agent to the site of the tumor, wherein the targeted delivery is conducted by applying a method according to any one of the claims 1 to 6.

8. A composition for the delivery of a bio-active molecule to a mammal comprising:
- a temperature sensitive carrier comprising a bio-active molecule
- an ultrasound active particle
- a suitable injection fluid

9. A composition according to claim 8, wherein the temperature sensitive carrier is a low temperature sensitive carrier with a release temperature between 39 and 44 degrees Celsius.

10. A composition according to any one of claims 7 and 8, wherein the bio-active molecule is a nucleic acid.
